# EUROPEAN PATENT APPLICATION

(11) **EP 0 967 291 A1**
(43) Date of publication of application: **29.12.1999**
(21) Application number: 99250176.7
(22) Date of filing: 04.06.1999
(51) Int. Cl.: C12Q 1/68

(54) **Method for parallel screening of allelic variation**

(30) Priority: 08.06.1998 US 93947
(71) Applicant: The Board of Trustees of The Leland S. Stanford Junior University, Palo Alto, CA 94304 (US)
(72) Inventor: Winzeler, Elizabeth, Menlo Park,CA 94025 (US); Richards, Dan, Los Altos,CA 94022 (US); Davis, Ronald, Palto Alto,CA 94301 (US)
(74) Representative: UEXKÜLL & STOLBERG

(57) **Abstract**

Parallel hybridization analysis is used to detection and analyze allelic variation between two closely related genomic nucleic acid samples. Nucleic acid samples from both sources are cleaved to generate short fragments. The fragments are end-labeled, and then hybridized to a high density oligonucleotide array. Hybridization patterns for the two samples are detected, normalized and compared. Those positions on the array that correspond to sequences with allelic variation between the two samples show decreased hybridization efficiency for one of the samples relative to the other. A map of allelic variation can be generated with this information, and used for genetic linkage analysis, determination of chromosomal regions having low diversity or high diversity, forensic studies, *etc.*

### ANNEX TO THE DESCRIPTION

SEQUENCE LISTING

<110> The Board of Trustees of the Leland Stanford Junior
      University

<120> Method for parallel screening of allelic variation

<130> P050173

<140>
<141>

<150> US 09/093,947
<151> 1998-06-08

<160> 2

<170> PatentIn Ver. 2.0

<210> 1
<211> 20
<212> DNA
<213> Artificial Sequence

<220>
<223> Description of Artificial Sequence: PCR primer PR49

<400> 1
aaacctaatg tgaccgtcgc
  20

<210> 2
<211> 21
<212> DNA
<213> Artificial Sequence

<220>
<223> Description of Artificial Sequence: PCR primer PR50

<400> 2
ccaaccatca agagaagaac c
  21

## Description

### STATEMENT AS TO FEDERALLY SPONSORED RESEARCH

This invention was made with Government support awarded by the National Institutes of Health, grants HG01633-01 and HG00185-01. The Government may have certain rights in this invention.

### INTRODUCTION

### Background

Genetic linkage maps show the relative locations of specific DNA markers along a chromosome. Any inherited physical or molecular characteristic that differs among individuals and is easily detectable in the laboratory is a potential genetic marker. DNA sequence polymorphisms are useful markers because they are plentiful and easy to characterize precisely. Many such polymorphisms are located in non-coding regions and do not affect the phenotype of the organism, yet they are detectable at the DNA level and can be used as markers. Examples include restriction fragment length polymorphisms (RFLPs), which reflect sequence variations in DNA sites or differences in the length of the product, which can be cleaved by DNA restriction enzymes, variable number of tandem repeat (VNTR) sequences, which are short repeated sequences that vary in the number of repeated units, single nucleotide polymorphisms (SNPs), and the like.

The "linkage" aspect of the map is a measure of how frequently two markers are inherited together. The closer the markers are to each other physically, the less likely a recombination event will fall between and separate them. Recombination frequency thus provides an estimate of the distance between two markers. The value of the genetic map is that an inherited trait can be located on the map by following the inheritance of a DNA marker present in affected individuals, but absent in unaffected individuals, even though the molecular basis for the trait may not yet be understood. Genetic maps have been used to find the exact chromosomal location of several important disease genes, including cystic fibrosis, muscular dystrophy, sickle cell disease, Tay- Sachs disease, fragile X syndrome and many others.

There is currently a substantial effort being put into sequencing the genome of a variety of organisms, including many viruses, bacteria, and eukaryotic organisms. Recent work has generated genetic maps of every human chromosome, and more refined maps are continuously being developed. This information makes it possible to perform whole genome screening for genetic mapping in a number of different species. When combined with statistical methods such as sib pair analysis, affected-pedigree-member analysis, or efficient Lod score analysis, whole genome screening is a powerful tool with which to identify genes.

One tool showing considerable promise for genome-wide analysis is the nucleic acid array, reviewed by Ramsay (1998) Nat. Biotech. **16**:40-44. These arrays contain dense collections of nucleic acids, either PCR products or oligonucleotides, usually of known sequence, that have been either synthesized or printed at fixed spatial locations on suitable substrates, such as nylon filters or glass slides. When labeled DNA or RNA samples are hybridized to the arrays, the abundance of specific sequences in solution can be quantitated based on the fluorescent or radioactive signal intensity at the position of the complementary probe. While recent interest has been directed toward the use of arrays for monitoring global gene expression, arrays can also be used for rapid detection of sequence variation.

An emerging class of marker for genetic analysis of the single nucleotide polymorphism, and other simple polymorphisms, *e.g.* deletions, double nucleotide polymorphisms, *etc*. SNPs are generally biallelic systems, that is, there are two alleles that a population may have for any particular marker. This means that the information content per SNP marker is relatively low when compared to microsatellite markers, which may have upwards of 10 alleles. SNPs also tend to be very population-specific; a marker that is polymorphic in one population may not be very polymorphic in another.

SNP markers offer a number of benefits that will make them an increasingly valuable tool. SNPs, found approximately every kilobase (see Wang *et al.* (1998) Science **280**:1077-1082), offer the potential for generating very high density genetic maps, which will be extremely useful for developing haplotyping systems for genes or regions of interest, and because of the nature of SNPs, they may in fact be the polymorphisms associated with the disease phenotypes under study. The low mutation rate of SNPs also makes them excellent markers for studying complex genetic traits.

In principle, any base that differs among allelic sequences could serve as a marker for linkage analysis. Single-base differences between allelic single copy sequences from two different haploid genomes have been estimated to occur about once per 300 bp in an outbred Western European population. This calculates to a total of about 10⁷ potential markers for linkage analysis per haploid genome. Only a tiny fraction of these nucleotide differences contribute to mapping using current methods. There is, therefore, substantial interest in developing new methods that utilize the available genomic information more efficiently and can provide information concerning multi-gene traits. Such methods could be valuable, not only for gene mapping, but also for genetic diagnosis and risk assessment. Allelic variation can be used for strain identification, in population genetics, linkage analysis and recombination studies.

### Relevant literature

The complete genome sequence of a number of organisms may be found at the National Center for Biotechnology Information, *http://www.ncbi.nlm.nih.gov/Entrez/ Genome/org.html.* The availability of sequences of genes of the human genome is discussed in Schuler (1996) Science **274**:540. The complete sequence of the genome of *S*. *cerevisiae* is available at several Intemet web sites, and is discussed in Goffeau *et al.* (1996) Science **274**:546.

A number of methods are available for creating microarrays of biological samples, such as arrays of DNA samples to be used in DNA hybridization assays. Exemplary are PCT Application Serial No. W095/35505, published December 28, 1995; U.S. patent no. 5,445,934, issued August 29, 1995; and Drmanac *et al., Science* **260**:1649-1652. Yershov *et al.* (1996) Genetics **93**:4913-4918 describe an alternative construction of an oligonucleotide array. The construction and use of oligonucleotide arrays is reviewed by Ramsay (1998) *supra.*

Methods of using high density oligonucleotide arrays are known in the art. For example, Milosavljevic *et al.* (1996) Genomics **37**:77-86 describe DNA sequence recognition by hybridization to short oligomers. The use of arrays for identification of unknown mutations is proposed by Ginot (1997) Human Mutation **10:**1-10.

Detection of known mutations is described in Hacia *et al.* (1996) Nat. Genet. **14:**441-447; Cronin *et al.* (1996) Human Mut.**7**:244-255; and others. The use of arrays in genetic mapping is discussed in Chee *et al.* (1996) Science **274**:610-613; Sapolsky and Lishutz (1996) Genomics **33:**445-456; *etc.* Shoemaker *et al.* (1996) Nat. Genet. **14:**450-456 perform quantitative phenotypic analysis of yeast deletion mutants using a parallel bar-coding strategy.

Quantitative monitoring of gene expression patterns with a complementary DNA microarray is described in Schena *et al.* (1995) *Science* **270**:467. DeRisi *et al.* (1997) Science **270**:680-686 explore gene expression on a genomic scale. Wodicka *et al.* (1997) Nat. Biotech. **15:**1-15 perform genome wide expression monitoring in *S. cerevisiae.*

### SUMMARY OF THE INVENTION

Methods are provided for detection and analysis of allelic variation between two closely related genomes, through parallel hybridization analysis. Detectable allelic variations may be substitutions, insertions or deletions of one or more nucleotides in length. A map of allelic variation can be generated with the subject methods, and used for genetic linkage analysis, determination of chromosomal regions having low diversity or high diversity, forensic studies, *etc.* By identifying the parental origin of DNA sequences in offspring, the locations of segregating loci can be determined in parallel. The subject methods have broad applicability to the analysis of variation and of the inheritance of multigenic or quantitative trait loci.

The provided methods utilize genomic DNA from two closely related sources. DNA samples from both sources are cleaved to generate short fragments. The fragments are end-labeled, and then hybridized to a high density oligonucleotide array. Hybridization patterns for the two samples are detected, normalized and compared. Those positions on the array that correspond to sequences with allelic variation between the two samples will show decreased hybridization efficiency for one of the samples relative to the other.

### BRIEF DESCRIPTION OF THE DRAWINGS

Figure 1 is a schematic illustrating the detection of allelic variation using high-density arrays.

Figure 2 is a comparison of hybridization patterns for two strains of *S. cerevisiae.*

Figure 3 is a schematic showing the inheritance of markers (3 chromosomes) in one tetrad from a cross between YJM789 and S96. The genotypes of the segregants are given in Table I.

Figure 4 is a schematic showing the inheritance of DNA in 10 segregants.

Figure 5 is a graph showing the probability of random segregation for the entire yeast genome.

Figure 6A, 6B and 6C are flow charts illustrating an exemplary data analysis for use with the subject methods.

### DESCRIPTION OF THE SPECIFIC EMBODIMENTS

Methods are provided for the rapid detection of allelic variation. Genomic DNA from two related samples are compared by hybridization to a high density DNA array. DNA samples from both sources are cleaved chemically or enzymatically to generate short fragments. The fragments are end-labeled, and then hybridized to a high density oligonucleotide array. Hybridization patterns for the two samples are visualized, normalized and compared. Probes that correspond to sequences with allelic variation between the two samples will show decreased hybridization efficiency for one of the samples relative to the other. A map of allelic variation can be generated with the subject methods, and used for genetic linkage analysis, determination of chromosomal regions having low diversity or high diversity, forensic studies, *etc.*

Knowledge of genetic variation is important for understanding why some people are more susceptible to disease or respond differently to treatments. Variation can also be used to determine which genes contribute to multigenic or quantitative traits such as increased yield or pest resistance in plants or for understanding why some strains of a microbe are exceptionally virulent. Genetic variation can also be employed for identification purposes, both in microbiology and in forensics, for studies of recombination, and in population genetics. Rapid and cost effective ways to analyze variation are clearly needed. The methods of the present invention allow genetic variation in any two isolates of a species to be scanned, mapped and scored directly and efficiently without allele-specific PCR, without creating new strains or constructs, and without knowing the specific nature of the variation.

One of the most important uses for variation is to map genetic differences within a species. The chromosomal location of such variation provides a means of identifying individuals, and of tracing inheritance for genetic mapping. The information derived from genetic mapping studies has a wide range of uses. For example, mapping is useful in agricultural species for tracing the genes associated with a particular phenotype. In human studies it is used for determining loci associated with traits such as disease predisposition.

Within a species, there are genetic sites that are polymorphic, *i.e.* within a population, more than one nucleotide (G, A, T, C) is found at a specific position. Allelic variation, as used herein, refers to polymorphisms in genomic DNA sequence between two individuals. Allelic variation may be substitution, addition or deletion of one or more nucleotides at a particular site. Frequently the detected variation will be a point mutation, or single nucleotide polymorphism. However, small deletions, additions, and multiple nucleotide variations are also detected.

The subject methods are also used to determine which genes or regions of genes are conserved, and which contain variable regions. Such information is useful, for example, in the design of vaccines where it is desirable to use epitopically conserved antigens; or in the choice of targets for drug screening. Alternatively, information about variable regions of the genome may indicate those loci that differ between pathogenic and non-pathogenic strains.

The source of genomic DNA is two strains or individuals from one species or two closely related species, where partial sequence information is available for one of the genomes. There should be a high degree of sequence identity between the two DNA samples, such as one would expect to find between individuals in a species. The percent of sequence identity will usually be at least about 99%, more usually at least about 99.5%, and may be at least about 99.9%, or higher.

The complete genome is used, or predetermined portions thereof, *e.g.* isolated chromosomes, messenger RNA fractions, BACs, YACs, cosmids, EST libraries, *etc.* One of the samples may, but does not necessarily, comprise a complete genome sequence, while the other sample comprises a pre-determined subpopulation of the genome. Where the complete genome is used in screening, it will preferably be obtained from a prokaryote, virus, or lower eukaryotes, *e.g.* fungi, protozoans, plants having a small genome, *etc.*

The sample complexity, *i.e.* the length of sequence that will be analyzed, will usually be less than about 10⁹ bp, preferably less than about 10⁸ bp, more preferably less than about 5 x 10⁷ bp in size, and may be less than about 1.5 x 10⁷ bp. A viral genome will usually be greater than 10³ nucleotides in length, while a bacterial genome will usually be greater than 10⁵ bp in length. Larger genomes, e.g. having a complexity of greater than about 10⁷ bp, or greater than about 10⁸ bp, may be separated into samples of lower complexity for analysis.

Partial sequence characterization of target regions in one of the samples is required. Dispersed nucleotide sequences of at least about 16 nucleotides, usually at least about 20 nucleotides and preferably at least about 25 nucleotides throughout the region to be analyzed are desirable. Known sequences may be dispersed throughout the genome, chromosome or locus of interest, usually spaced not more than about 10,000 nucleotides apart, more usually not more than about 1000 nucleotides apart, and preferably not more than 500 nucleotides apart.

A number of organisms have sufficient sequence information to meet these requirements, including organisms with complete known genome sequences, *e.g. Aquifex aeolicus; Archaeoglobus fulgidus; Bacillus subtilis; Borrelia burgdorferi; Escherichia coli; Haemophilus influenzae; Helicobacter pylori; Methanobacterium thermoautotrophicum; Methanococcus jannaschii; Mycoplasma genitalium; Mycoplasma pneumoniae; Saccharomyces cerevisiae; Synechocystis PCC6803;* and organisms with substantial sequence and mapping information known, *e.g. Arabidopsis thaliana; Caenorhabditis elegans; Drosophila melanogaster; Homo sapiens; Leishmania major*, *Mus musculus; Oryza sativa; Saccharomyces cerevisiae; Zea mays, etc.*

The two DNA samples are prepared initially in accordance with conventional methods, *e.g.* lysing cells, removing cellular debris, separating the DNA from proteins, lipids or other components present in the mixture and then using the isolated DNA for cleavage. See Molecular Cloning, A Laboratory Manual, 2nd ed. (eds. Sambrook *et al.)* CSH Laboratory Press, Cold Spring Harbor, NY 1989. Usually, at least about 0.5 µg of DNA will be employed, more usually at least about 5 µg of DNA, while less than 50 µg of DNA will usually be sufficient.

The nucleic acid samples are cleaved to generate probes. It will be understood by one of skill in the art that any method of random cleavage will generate a distribution of fragments, varying in the average size and standard deviation. Usually the average size will be at least about 12 nucleotides in length, more usually at least about 20 nucleotides in length, and preferably at least about 35 nucleotides in length. Where the variation in size is great, conventional methods may be used to remove the large and/or small regions of the fragment population.

It is desirable, but not essential to introduce breaks randomly, with a method which does not act preferentially on specific sequences. Preferred methods produce a reproducible pattern of breaks. Methods for introducing random breaks or nicks in nucleic acids include reaction with Fenton reagent to produce hydroxyl radicals and other chemical cleavage systems, integration mediated by retroviral integrase, partial digestion with an ultra-frequent cutting restriction enzymes, partial digestion of single stranded with S1 nuclease, partial digestion with DNAse I in the absence or presence of Mn⁺⁺, *etc.*

The fragmented nucleic acid samples are denatured and labeled. Labeling can be performed according to methods well known in the art, using any method that provides for a detectable signal either directly or indirectly from the nucleic acid fragment. In a preferred embodiment, the fragments are end-labeled, in order to minimize the steric effects of the label. For example, terminal transferase may be used to conjugate a labeled nucleotide to the nucleic acid fragments. Suitable labels include biotin and other binding moieties; fluorochromes, *e.g.* fluorescein isothiocyanate (FITC), rhodamine, Texas Red, phycoerythrin, allophycocyanin, 6-carboxyfluorescein (6-FAM), 2',7'-dimethoxy-4',5'-dichloro-6-carboxyfluorescein (JOE), 6-carboxy-X-rhodamine (ROX), 6-carboxy-2',4',7',4,7-hexachlorofluorescein (HEX), 5-carboxyfluorescein (5-FAM) or N,N,N',N'-tetramethyl-6-carboxyrhodamine (TAMRA), and the like. Where the label is a binding moiety, the detectable label is conjugated to a second stage reagent, *e.g.* avidin, streptavidin, *etc.* that specifically binds to the binding moiety, for example a fluorescent probe attached to streptavidin. Incorporation of a fluorescent label using enzymes such as reverse transcriptase or DNA polymerase, prior to fragmentation of the sample, is also possible.

Each of the labeled genome samples is separately hybridized to an array of oligonucleotide probes. Hybridization of the labeled sequences is accomplished according to methods well known in the art. Hybridization can be carried out under conditions varying in stringency, preferably under conditions of high stringency, *e.g.* 6X SSPE, 65°C, to allow for hybridization of complementary sequences having extensive homology, usually having no more than one or two mismatches in a probe of 25 nucleotides in length, *i.e.* at least 95% to 100% sequence identity.

High density microarrays of oligonucleotides are known in the art and are commercially available. The sequence of oligonucleotides on the array will correspond to the known target sequences of one of the genomes, as previously described. Arrays of interest for the subject methods will generally comprise at least about 10³ different sequences, usually at least about 10⁴ different sequences, and may comprise 10⁵ or more different sequences. The length of oligonucleotide present on the array is an important factor in how sensitive hybridization will be to the presence of a mismatch. Usually oligonucleotides will be at least about 12 nt in length, more usually at least about 15 nt in length, preferably at least about 20 nt in length and more preferably at least about 25 nt in length, and will be not longer than about 35 nt in length, usually not more than about 30 nt in length.

Methods of producing large arrays of oligonucleotides are described in U.S. Patent no. 5,134,854 (Pirrung *et al.),* and U.S. Patent no. 5,445,934 (Fodor *et al.)* using light-directed synthesis techniques. Using a computer controlled system, a heterogeneous array of monomers is converted, through simultaneous coupling at a number of reaction sites, into a heterogeneous array of polymers. Alternatively, microarrays are generated by deposition of pre-synthesized oligonucleotides onto a solid substrate, for example as described in International Patent application WO 95/35505.

Microarrays can be scanned to detect hybridization of the labeled genome samples. Methods and devices for detecting fluorescently marked targets on devices are known in the art. Generally such detection devices include a microscope and light source for directing light at a substrate. A photon counter detects fluorescence from the substrate, while an x-y translation stage varies the location of the substrate. A confocal detection device that may be used in the subject methods is described in U.S. Patent no. 5,631,734. A scanning laser microscope is described in Shalon *et al.* (1996) Genome Res. **6:**639. A scan, using the appropriate excitation line, is performed for each fluorophore used. The digital images generated from the scan are then combined for subsequent analysis. For any particular array element, the ratio of the fluorescent signal from one Nucleic acid sample is compared to the fluorescent signal from the other Nucleic acid sample, and the relative signal intensity determined.

Methods for analyzing the data collected by fluorescence detection are known in the art. Data analysis includes the steps of determining fluorescent intensity as a function of substrate position from the data collected, removing outliers, *i.e.* data deviating from a predetermined statistical distribution, and calculating the relative binding affinity of the targets from the remaining data. The resulting data may be displayed as an image with the intensity in each region varying according to the binding affinity between targets and probes.

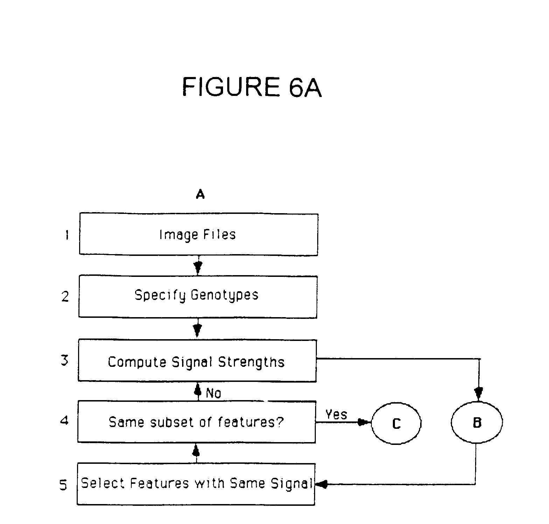

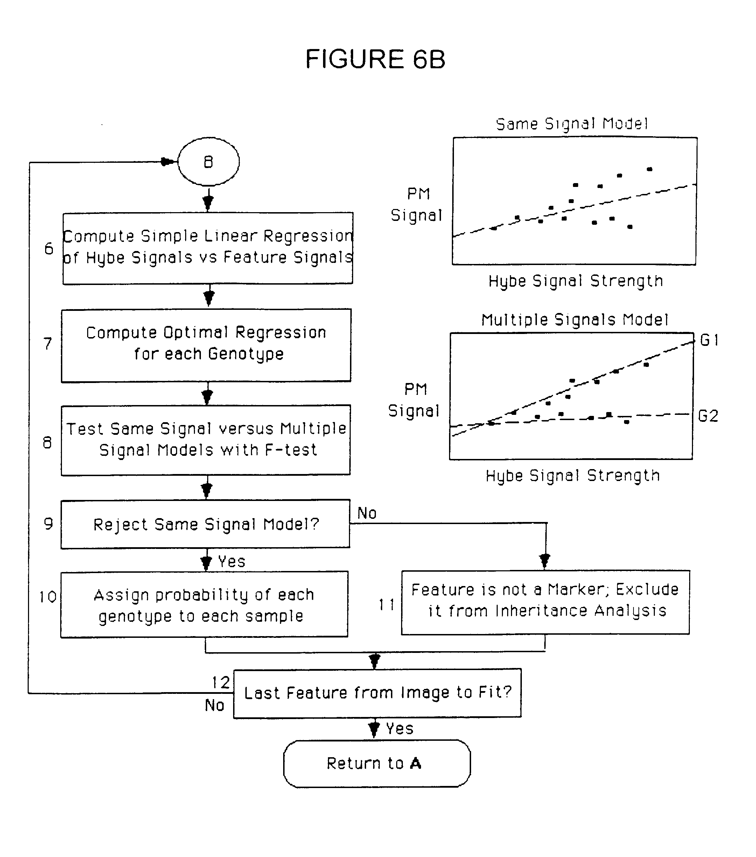

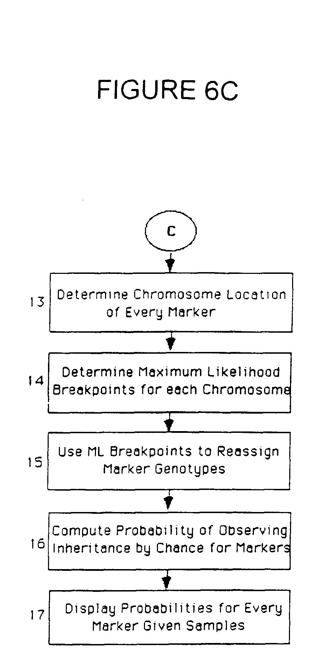

The images from the two or more genome samples from the two strains, or progeny from crosses of the two strains are compared to determine feature signals showing a bimodal distribution pattern, *i.e.* that detect allelic variation. A flow chart of the data analysis process is provided in Figure 6A, 6B and 6C. Referring to FIG. 6A (steps 1 and 2), the system is initialized by requesting the user to enter the names of the sample CEL files and their genotypes (if known). The CEL files contain the quantitated feature intensities from the scanned images. The feature intensities from the CEL files are adjusted with a monotonic, variance-stabilizing transformation. At step 3, the overall signal strength of each image are estimated as the mean of a subset (initially all) of the features.

Next, the expected signal response for each feature is determined using the data from the CEL files. First (step 6), for each feature, a single regression line is fit to the overall signal strengths of the images (x axis) and their corresponding adjusted feature intensities (y axis). This determines the expected signal response and variance for the feature given the signal strength of the image. However, this assumes that the signal response is the same for all samples *(i.e.* there is only one genotype). Next, separate lines are fit for each genotype in parallel (step 7) to model the expected signal responses if there are actually multiple genotypes. Samples whose genotypes are unknown are assigned to the genotype that minimizes the variance of the resulting fits. An F-test is used to distinguish between these models. If the same signal model is rejected, genotypes are assigned to each sample whose genotype is unknown along with the probability that the genotype is correct. This probability is computed using the expected signals and variances from the regression fits at the sample's overall signal strength based on a t-distribution. For example, Pr(G1)=P(G1)/(P(G1)+P(G2)) where G1 is the assigned genotype, G2 is the other genotype, and P(X) is the probability of observing the signal given the expected signal and variance for genotype X. The overall signal strengths are re-estimated using only the features that have the same signal response, regardless of genotype, and this process is repeated until this subset of features does not change significantly.

Then the chromosome location of every feature corresponding to a marker is determined (step 13). Any features that appear more than once in the genome are excluded from the analysis. Next, the meiotic breakpoints are determined for each sample. The marker genotype probabilities along each chromosome are used to determine these sites by maximum likelihood (step 14). Additional breakpoints are added only if they substantially increase the log likelihood ratio, which tests the model containing an additional breakpoint against the current one.

If the data are to be used for mapping purposes, the breakpoints are used to reassign the inherited genotypes, eliminating noise at step 15. Then, the genotypes for each marker from all of the samples are compared. The probability of observing the genotypes by chance is computed from the given genotypes. This information is then displayed by chromosome to indicate which regions of the genome were inherited non-randomly.

Genetic linkage markers are polymorphic sequences distributed throughout a genome. Using the subject methods, polymorphisms are detected as a sequence difference between the compared genomes. A wide variety of polymorphic markers may be identified for any given genome. The subject methods may be used in mapping genes by use of family studies, segregating tetrads, pairs of relatives that have a genetically influenced trait of interest, *etc.* "Affected relative pair" methods are useful when the penetrance of the allele that confers the trait is low or age-dependent, or when the trait is multigenic or quantitative, *e.g.* height and build. Disease-susceptibility genes are particularly relevant. By determining where on the genetic map a small set, including two, of "affected" relatives have inherited identical sequences from a common source, and disregarding other family members, a highly efficient strategy for extracting linkage information from a pedigree is provided. The resulting identity-by-descent maps from multiple pairs of similarly-affected relatives can be combined and the composite map searched for loci where genotypic concordance between affected relatives occurs more frequently than would be expected by chance. With a sufficiently large number of affected relative pairs, such an analysis can reveal the positions of genes that contribute even a slight susceptibility to the trait. The procedure may also find wide application in routine screening for shared genetic risks in families.

The subject methods find application in following segregation of traits associated with breeding of plants and animals, the association of particular regions in the genomic map with particular traits, especially traits associated with multiple genes, the transmission of traits from ancestors or parents to progeny, the interaction of genes from different loci as related to a particular trait, and the like. While only two sources may be involved in the comparison, a much larger sampling may also be used, such as 20 or more sources, where pairwise comparisons are made between the various sources. Relationships between the various sources may vary widely, *e.g.* grandparents and grandchildren; siblings; cousins; and the like.

The subject methods may also be used for the ordered mapping of genomic libraries. Typically, the term "genomic library" is defined as a set of sequence fragments derived from one or more genome molecules. Such molecules may be whole chromosomes, subsets thereof, plasmids, or other similar large polynucleotides. Specifically, the methods of the present invention are useful for mapping high molecular weight polynucleotides including chromosomal fragments, cosmids, yeast artificial chromosomes (YACs), *etc.*

Mapping techniques typically involve the identification of specific genetic markers on individual nucleic acid fragments from a genomic library. Comparison of the presence and relative position of specific markers on fragments generated by different cleavage patterns allows for the assembly of a contiguous genomic map, or "contig". Methods of genomic mapping are provided, using the allelic variant detection methods already described. Polymorphic sites are identified on the individual fragments of a genomic library using the methods described above. Sites that demonstrate a bimodal distribution pattern are used as genetic markers, and a contig of the particular library is then assembled. The exact sequence of variants can be determined by various methods known in the art, e.g. PCR amplification followed by sequence determination of the amplification product.

When repeated on separate fragments from the library, each fragment will generally produce a distinctive hybridization pattem. These hybridization patterns may be compared with hybridization patterns from differentially generated fragments. Where a specific marker is present in both fragments, it is an indication of potential overlap between the fragments. Two fragments that share several of the same markers, *i.e.* overlapping fragments, will show similar hybridization patterns on the oligonucleotide array. The greater the similarity or correlation between two fragments, the higher the probability that these fragments share an overlapping sequence. By correlating the hybridization pattem of each fragment in the library against each other fragment in the library, a single contiguous map of the particular library can be constructed.

In practice, each fragment is correlated to each other fragment, and a correlation score is given based upon the number of probes which cross-hybridize with a marker of both the first and second fragment. High scores indicates high overlap. For example, the comparison of two identical sequences would produce a correlation score of 1. Similarly, sequences sharing no overlapping sequence would ideally produce a correlation score of 0. In practice, sequences that do not overlap will generally have correlation scores above zero, due to potential non-specific hybridizations, *e.g.* single base mismatches, background hybridization, duplicated sequences, which may provide some baseline correlations between otherwise unrelated fragments. As a result, a cutoff may be established below which correlation scores are not used. The precise cutoff may vary depending upon the level of nonspecific hybridizations for the particular application.

The methods described herein are useful in a variety of applications. For example, as is described above, these methods can be used to generate ordered physical maps of genomic libraries, as well as genetic linkage maps which can be used in the study of genomes of varying sources. The mapping of these genomes allows further study and manipulation of the genome in diagnostic and therapeutic applications, *e.g.* gene therapy, diagnosis of genetic predispositions for particular disorders and the like.

In addition to pure mapping applications, the methods of the present invention may also be used in other applications. For example, the methods described herein are used in the identification of the source of a particular sample. This application would include forensic analysis to determine the origin of a particular tissue sample, such as analyzing blood or other evidence in criminal investigations, paternity investigations, *etc.* Additionally, these methods can also be used in other identification applications, for example, taxonomic study of plants, animals, bacteria, fungi, viruses, *etc.* This taxonomic study includes determination of the particular identity of the species from which a sample is derived, or the interrelatedness of samples from two separate species. Where a hybridization pattem from both the sample and the source are identical or highly similar, it is indicative that the sample was derived from the source. Where the sequences captured from the sample and known source share a large number of identical sequences, it is indicative that the sample is related to the known source. However, where the sample and source share few like sequences, it is indicative of a low probability of interrelation.

Precise levels of interrelation to establish a connection between source and sample will typically be established based upon the interrelation which is being proved or disproved, the identity of the known source, the precise method used, and the like. Establishing the level of interrelation is well within the ordinary skill in the art. For example, in criminal investigations, a higher level of homology between sample and known source sequences will likely be required to establish the identity of the sample in question. Typically, in the criminal context, interrelation will be shown where there is greater than 95% marker identity, preferably greater than 99%, and more preferably, greater than 99.9% identity. For other identification applications, interrelation between sample and known source may be established by a showing of greater than 50% identity, and typically greater than 75% identity, preferably greater than 90% identity, and more preferably greater than 95 to 99% identity.

For convenience, kits may be supplied which provide the necessary reagents in a convenient form and together. For example kits could be provided that include chips containing an appropriate microarray for the subject to be analyzed, terminal transferase, DNAse I, biotin labeled nucleotides, and/or fluorochrome labeled avidin. Other components such as automated systems for determining and interpreting the hybridization results, software for analyzing the data, or other aids may also be included depending upon the particular protocol which is to be employed.

### EXPERIMENTAL

### Detection of Allelic Variation in S. cerevisiae

*Strain Selection.* To maximize the amount of allelic variation that could be detected, two distantly-related *S. cerevisiae* strains, S96 *(MA Ta ho lys5 mal*)*,* isogenic with S288c, and YJM789 *(MA Tα ho::hisG lys2 pdr5 MAL)* were chosen for this study. The *S. cerevisiae* genome sequence is from strain S288c and 88% of the S288C genome is derived from EM93, which was isolated from a rotting fig near Merced, Califomia in 1938. YJM789 is isogenic with YJM145, a segregant of a clinical isolate of *S. cerevisiae.* YJM145 has been characterized genetically, and the ultimate source of its parent (human lung) differs significantly from that of S288c in that the strains were isolated from different environments, at different times and in different geographic locations. S288c and YJM789 were considered to be unrelated, and therefore likely to exhibit considerable allelic variation.

To determine the frequency of allelic variation in YJM789, a library of YJM789 genomic DNA was constructed and partially sequenced. Genomic DNA was isolated from strain YJM789 and sheared to 1000-basepair insert sizes using a re-circulating point-sink flow shearing device (Oefner *et al.* (1996) *Nucleic Acids Res* **24**, 3879-86). Fragments were cloned into an M13 sequencing vector and the sequence was determined for 696 clones using dye-primer chemistry in cycle-sequencing reactions on ABI 377 sequencing machines (Dietrich *et al.* (1997) *Nature* **387**:78-81). The sequences were called using *phred* basecaller software (see *http://chimera,biotech.washington.edu/UWGC/tools/phred.htm*), which produces a quality measurement for each base (-10 x log10 (probability of an error)). Using this quality measurement, 122258 bases were sequenced with > 99% confidence. The YJM789 sequences were compared to the fully sequenced strain of S. *cerevisiae* using the *cross_match* program (see *http.//chimera.biotech. washington.edu/UWGC/tools/ phrap.htm*). Discrepancies between the YJM789 and S288c sequences were then classified by quality and assigned into coding and non-coding regions using the phred basecaller. In most cases, since only a single trace was available and no alignments were performed, regions of the traces that did not show high quality were excluded from the analysis.

When high quality sequence (>99.7% accurate) from YJM789 was aligned with that of S288c, 466 cases of allelic variation were observed with a frequency of one every 160 bases. Most were single-base pair polymorphisms, but small insertions and deletions were also observed. Large deletions were not readily identified by this shotgun sequencing approach because of the difficulty associated with aligning the sequence fragments using automated methods. A small bias (10%) toward non-coding regions was observed. 288 of the 466 cases of allelic variation in sequences with >99.97 accuracy were from coding regions (61%). 8.637 Mb of the estimated 13.2 Mb yeast genome is annotated as coding sequence by SGD (65%). These data suggested that if some fraction of the existing allelic variation could be rapidly and reproducibly detected, a dense genome-wide genetic map could be constructed.

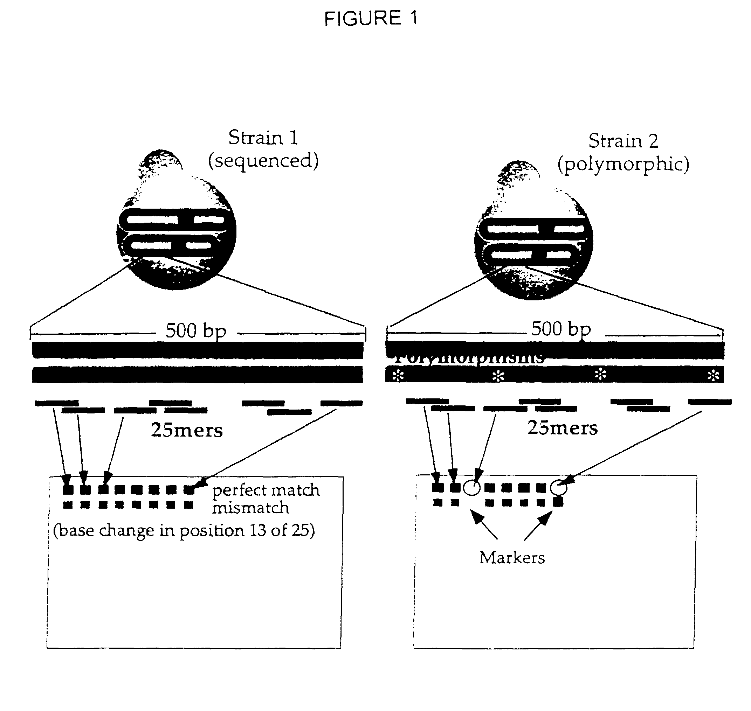

*High-density oligonucleotide arrays.* Commercially available high-density arrays containing a large number of oligonucleotide probes from genomic DNA sequence have been designed and used to monitor genome-wide gene expression in yeast. For oligonucleotide probes, hybridization is dependent on the absence of mismatches in the corresponding target sequence (Conner *et al.* (1983) *Proc Natl Acad Sci U S A* 80:278-82), and thus it was hypothesized that these arrays could serve in the rapid detection of allelic variation in yeast (Figure 1). These arrays contain 20 or more 25mer oligonucleotide probes derived from the sequence of each annotated open reading frame in the yeast genome.

Figure 1 shows a schematic for detection of allelic variation using high-density arrays. A minimum of 20 25-base oligonucleotide probes was chosen from yeast genomic sequence for every annotated open reading frame in the yeast genome. Probes were arranged on the array in a way that generally reflected their position in the genome. All probes were from predicted coding regions with a bias toward the 1000 bases at the 3' ends of genes. When YJM789 DNA fragments containing polymorphic regions (*) are hybridized to the array localized decreases in signal intensity are observed if a probe complementary to this region is found of the array.

In addition to probes designed to be perfectly complementary to regions of yeast coding sequence (designated perfect match or PM probes), probes containing a single base mismatch (MM) in the central position of the oligonucleotide were also synthesized in a physically adjacent position. The mismatch probes serve as local background and non-specific hybridization controls (Wodicka *et al.* (1997) *Nature Biotechnology* **15**:1359-1 367).

The probes were synthesized in a spatially-addressed fashion using a combination of photolithography and solid-phase chemistry (Fodor *et al.* (1991) *Science* **251**:767-73), on a series of five 1.64 cm² arrays. Each array contains more than 65,000 synthesis features, with each feature consisting of more than 10⁷ copies of the specific oligonucleotide probe. The collection of five arrays contains a total of 157,112 different 25mer probe pairs.

Excluding the rDNA and *CUP1* repeats, the largest gap is 41,325 bases wide at position 510,000 on Chromosome XII. This region contains three tandem repeats of the *ASP3* gene and an adjacent gene of unknown function, a region of ribosomal DNA and a Ty-1 element. Probes complementary to this region are present on the array but were ignored in the analysis, as were all non-unique probes. Though some probes spatially overlap one another, the collection of five arrays covers 21.8% of the non-repetitive regions of the yeast genome.

*Detecting allelic variation using high density* oligonucleotide *arrays.* Due to the high-degree of genomic coverage (22%), it was expected that a significant fraction of the allelic variation in YJM789 could be detected using the arrays. To test this, genomic DNA from S96 and YJM789 was isolated, fragmented and biotin-labeled.

Yeast cells were grown in YEPD to late log phase at 30°C. Genomic DNA was purified using Qiagen genomic DNA 100 µg columns according to the manufacturer's protocol. Zymolyase and protease digestion times were extended from 30 to 45 minutes. DNA was re-suspended in 400 µl TE, reprecipitated, and re-suspended in 30µl deionized H₂O. Yeast genomic DNA (10 µg ) was digested in 0.15 Units DNAse I (Gibco BRL PCR grade) in 1 X One-Phor-All buffer (Pharmacia) containing 1.5 mM CoCl₂ for 5 minutes at 37°C. The reaction was stopped by heating the samples to 100°C for 15 minutes. Digestion was checked by examining 1 µl of the reaction product on a 2% agarose gel containing 1:10000 SYBR-II green (Molecular Probes, Eugene, OR). The procedure was repeated if the majority of the product was not digested to a size of less than 100 bases (it was observed that the reproducibility of the reaction was highly sensitive to contaminants in the DNA preparation, such as EDTA). The DNA fragments were labeled by incubating the samples with 25 U terminal transferase (Boehringer Mannheim) and 1 nmole Biotin-N6-ddATP (NEN) for one hour at 37°C. The entire sample was hybridized to the array in a 200 µl volume containing 6X SSPE (Accugene), 0.005 % Triton-X 100 detergent, 20 µg fragmented denatured Salmon Sperm DNA (Gibco-BRL) and 1 nmole of a 3'-biotin control oligonucleotide that hybridizes to the border features on the array.

Samples were heated to 100°C for 10 minutes, and then cooled on ice before being applied to the array. Samples were hybridized for 2 hours at 42°C. The arrays were washed, stained with phycoerythrin-streptavidin (Molecular Probes) and scanned at an emission wavelength of 560 nm at 7.5 µM resolution using an Affymetrix GeneChip Scanner as previously described (Wodicka *et al., supra*.)

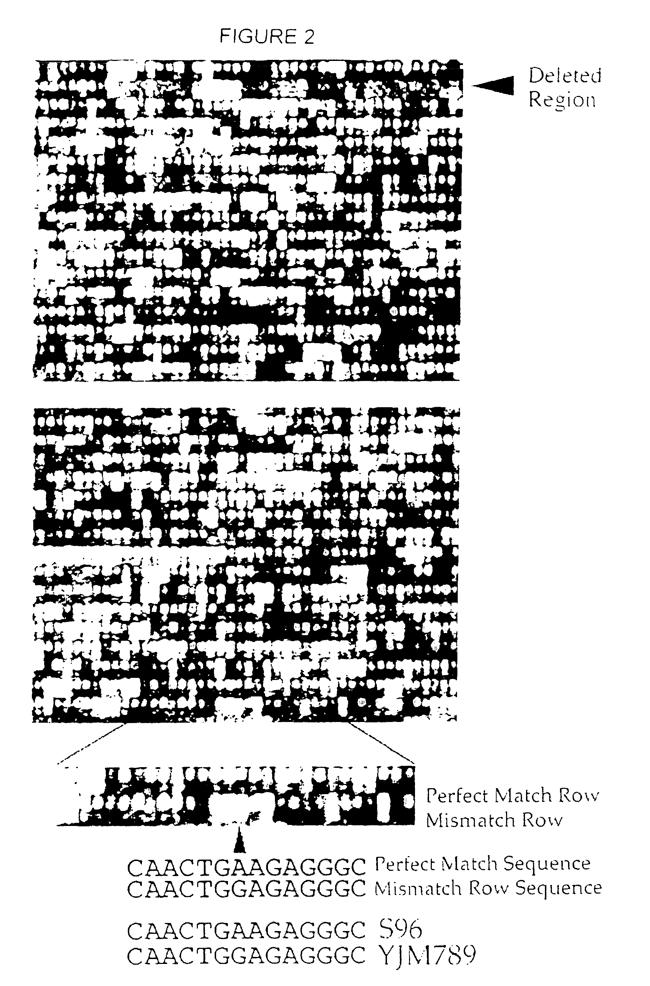

After hybridization, the arrays were washed, stained with a phycoerythrin-streptavidin conjugate and scanned with a laser confocal scanning device that detects and records the amount of fluorescence at approximately three million physical locations. Scanned images of arrays hybridized with S96 and YJM789 DNA were collected. For illustration, the images from the arrays hybridized with S96 and YJM789 DNA were colored red and green, respectively. The two images were electronically superimposed on one another and a portion of the array is shown in Figure 2. Regions in yellow indicate probes that hybridized roughly equally to genomic DNA from the two parental strains, while regions in red are locations of allelic variation where S96 DNA hybridized to a greater extent than DNA from YJM789. Isolated red spots covering one to five probe features are caused by short polymorphic stretches in the YJM789 sequence at these elements on the array. A few large deletions were also evident. Some green spots, usually in the mismatch (MM) row, may be due to YJM789 DNA hybridizing more strongly with the S96 mismatch sequence. An example of this sort is shown in Figure 2. The fact that the two scanned images can be superimposed demonstrates the reproducibility of the experiment, a feature critical to the analysis of a large number of scanned images obtained with different DNA samples and generated at different times using different arrays.

Figure 2 is a comparison of hybridization patterns for two strains of *S. cerevisiae.* DNA samples from YJM789 and S96 were labeled and hybridized to two separate sets of arrays. The array hybridized with DNA from S96 was colored red digitally; the image from the array hybridized with YJM789 DNA was colored green and the two scanned images were merged. Only a fraction of the array is shown. Probes which hybridized S96 DNA more efficiently than YJM789 DNA are red while probes that hybridize to both DNA types with equal intensity are yellow. Some yellow features are brighter than others, because some oligonucleotides hybridize more efficiently. These differences in hybridization signal intensity are reproducible and do not adversely affect the analysis. The figure close-up shows a region in which one of the mismatch features is bright green. Shotgun sequencing of YJM789 demonstrated that the actual sequence of YJM789 was complementary to the sequence of the oligonucleotide in the mismatch row and not to that in the perfect match row.

To collect a robust set of markers, two additional hybridizations of each parental strain DNA sample were performed and the hybridization intensity for each probe in the scanned image was quantitated. Grids were aligned to the scanned images using the known feature dimensions of the array. The hybridization intensities for each of the elements in the grid were determined using the 75th percentile method in the Affymetrix GeneChip^{a} software package.

Markers were selected recursively by analyzing the scanned images of 20 array sets hybridized with different DNA samples (3 samples from each parental strain, and 14 samples from haploid progeny derived from sporulation of a YJM789/S96 diploid, described below and in Table 1 using software written for this purpose.

The overall array hybridization intensity (/) for each hybridization (20 altogether) was determined by calculating the mean PM signal intensity using all features that showed little normalized variation across all hybridizations (non-markers), determined recursively as described below. Then for each feature on the array, a regression line of PM on I for each hybridization was determined by the least squares method first under the null hypothesis that the S96 and YJM789 samples had the same response, and then under the alternative hypothesis that the S96 samples had a higher signal than the YJM789 sample *(i.e.* a marker). The models were compared with the F-test and the identical signal model was rejected in favor of a marker with alpha=0.01.

3808 of the probes on the array were estimated to have a 99% or higher probability of being a marker based on their exhibiting a consistent bimodal distribution for all hybridizations. These markers were expected to be from probes whose complementary sequence is completely absent in YJM789 or whose complementary sequence contained a base change in the central region of the oligonucleotide probe. 25% of the polymorphisms detected by sequencing and having a corresponding probe on the array were found in the set of 3808 markers. In these cases, the base change was almost invariably in the central 10 bases of the complementary 25mer probe.

Excluding the rDNA repeat on Chromosome XII, the average marker spacing for this set of 3808 markers was 3510 bp. 14 gaps were observed with the largest gaps (59 kb) centered near position 150400 on Chromosome III. Gaps were often found near regions with low probe density, for example, near repeated elements in the genome but in some cases, probe density was adequate, suggesting that the gap might be due to a high level of conservation or to a recent common origin of the region between the two strains.

*Meiotic recombination breakpoints and segregation of markers.* To determine whether the set of chosen probes constituted a robust set of markers usable for linkage analysis, meiotic inheritance was examined. An S96/YJM789 diploid was sporulated and DNA from four segregants of one tetrad was isolated and hybridized to the arrays. The data was analyzed and a score (S96 or YJM789) and a confidence value, *p,* was assigned to each of the 3808 markers for each hybridization.

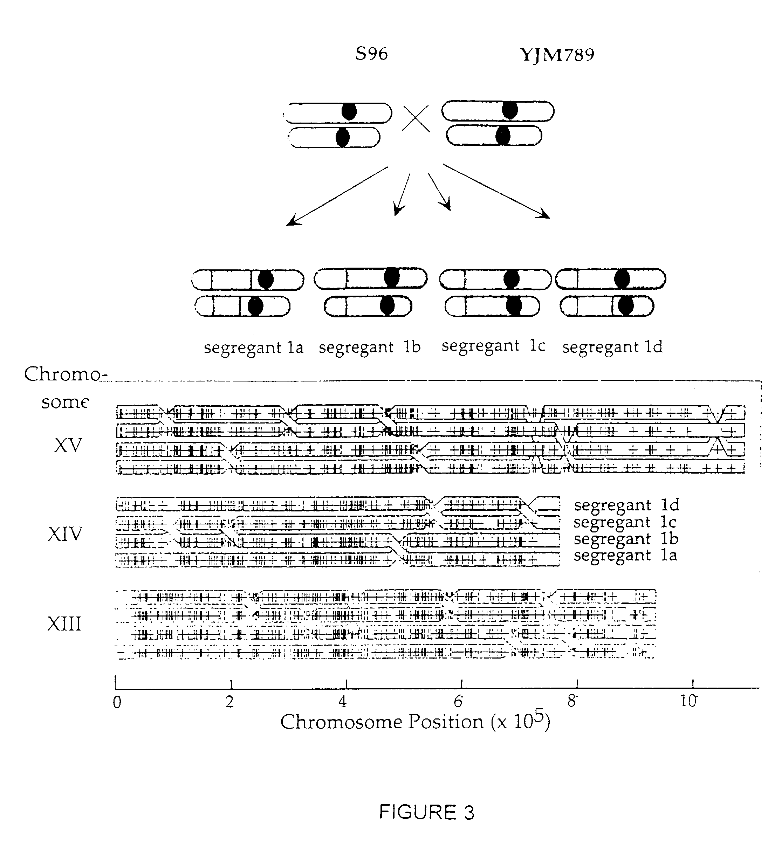

It was expected that half the markers would be scored as having an S96 origin and half would be scored as YJM789; that in most cases each marker would segregate with a ratio of 2:2 in the four segregants; and that crossovers would be observed about once per every 290 kb (1 cM = 2.9 kb for chromosomes XIII, XIV and XV). The locations of the markers, and each marker's score (S96 or YJM789) are shown for three chromosomes (Figure 3).

Figure 3 shows the inheritance of markers (3 chromosomes) in one tetrad from a cross between YJM789 and S96. The location of the marker on the chromosome is indicated below. Markers that exhibit the YJM789 hybridization pattern are colored red while markers that exhibit the S96 hybridization pattern are colored green. The probable locations of cross-over events are shown for each segregant. The genotypes of the segregants are given in Table I.

For the three chromosomes (about 2.8 million bases), 21 cross-overs were observed at an average of 1 per 268 kb, close to the expected value (1 per 290 kb). For the entire genome 97 cross-overs were observed (90 expected).

1051 of the markers had a high *p* value (less than 5% probability of an error) for all four segregants. *p* is the probability of observing a signal for a particular marker using a t distribution, based on the estimated variance and expected signal of that feature for all hybridizations examined. For this set, the number of markers scored as having an S96 origin was approximately equal to those having an YJM789 origin (2080 were YJM789 and 2124 were S96 in origin). Of these, 95.9% segregated 2:2. For this group, some of the markers segregating 3:1, or 4:0 are probably the result of non-reciprocal recombination events. Gene conversion occurs in yeast at frequencies ranging from 0.5 % to 30% per locus per tetrad, in agreement with these results.

For the remaining markers, *p* for at least one of the segregants was too low to estimate the frequency of gene conversion. The average number of markers segregating 2:2, for the entire set of 3808 markers for the tetrad was 78.3%. These data suggest that the probability of mis-scoring a marker for the set of data examined here was approximately 5%, but that the probability that a marker will be mis-scored for a particular hybridization is strongly correlated with its *p* value and is thus predictable. In studies of single marker events such as gene conversion, or for high-resolution mapping, increased confidence in individual marker quality could be obtained by repeating those hybridizations that gave overall low confidence scores for the set of markers. Regardless, a very clear inheritance pattern was discerned, indicating that linkage analysis could be performed using this set of markers.

*Mapping multiple simple traits with high-density arrays.* The YJM789 strain and the S96 strain are phenotypically distinguishable. It was predicted that the genomic regions encoding the molecular bases for these differences could be identified by hybridizing DNA from segregants of a cross between the two strains to the array and analyzing the inheritance of alleles. YJM789 *(MAT*α) carries a mutation in the *lys*2 gene on Chromosome II and contains an insertion in the homothallic mating type locus *(ho::hisG)* on Chromosome IV. S96 carries a mutation in the *lys5* gene (Chromosome VII) and a deletion in the homothallic mating-type-switching locus *(ho)* that is distinguishable by PCR from the mutation carried by YJM789. The *ho* alleles of YJM789 and S96 were scored by performing PCR using primers PR49 (SEQ ID NO:1) (5' AAACCTAATGTGACCGTCGC 3') and PR50 (SEQ ID NO:2) (5' CCAACCATCAAGAGAAGAACC 3') on genomic DNA, and checking the size of the products by agarose gel electrophroresis.

In addition, relative to S96, YJM789 is hyper-sensitive to multiple drugs, including cycloheximide. Cycloheximide hypersensitivity segregated 2:2 in 99 tetrads of a cross between S96 and YJM789 indicating that a single locus is responsible for the phenotype. Analysis of other crosses between the YJM789 parent strain and an S288c background strain mapped this cycloheximide hypersensitive mutation to between *ade2* and *his3.* This map location suggested allelism with *pdr5,* one of the S. *cerevisiae* multidrug resistance gene homologs.

A test cross was performed between YJM789 *(MA Tα lys2 ho:hisG cyh)* and S96 *(MATa lys5 ho).* After mating, the S96/YJM789 diploid was sporulated and the segregants of 99 tetrads were classified. Yeast strains were routinely grown in YEPD medium; sporulation medium and defined medium for scoring auxotrophs were prepared as previously described (Sherman *et al. Methods in Yeast Genetics: Laboratory Manual* (Cold Spring Harbor Laboratory, Cold Spring Harbor, NY, 1974)). Segregants were complementation tested to distinguish *lys2* from *lys5.* Cycloheximide sensitivity was scored by inability to grow on YEPD plates containing 0.5 pg/ml cyclohexamide (added after autoclaving).

Of the 396 segregants examined, 17 segregants were identified that were MATα *lys2 LYS5 ho cyh.* DNA from some of these segregants (ten) was prepared and hybridized to the arrays and the hybridization patterns were analyzed until all five loci could be unambiguously assigned to a specific genetic interval.

The loci could have been mapped using any segregant as long as the genotype was known, however, segregants with similar phentypes were chosen to simplify the analysis.. The probability of an interval segregating 10 to 0 randomly (a false positive) was estimated to be about 40% for each outcome. No false positives were observed with 10 segregants and therefore no additional hybridizations were performed. This conservative estimate of probability, which does not take into account recombination hotspots, or interference, was calculated by dividing the genome size (12 Mb), by the average interval, (29 kb for 10 segregants using 1 cM = 2.9 kb for yeast) and then multiplying by the probability of 10 events having the same outcome. In general, up to 13 segregants (or more if the trait is non-Mendelian) may need to be examined to have a 95% probability of identifying a single region as responsible for a trait.

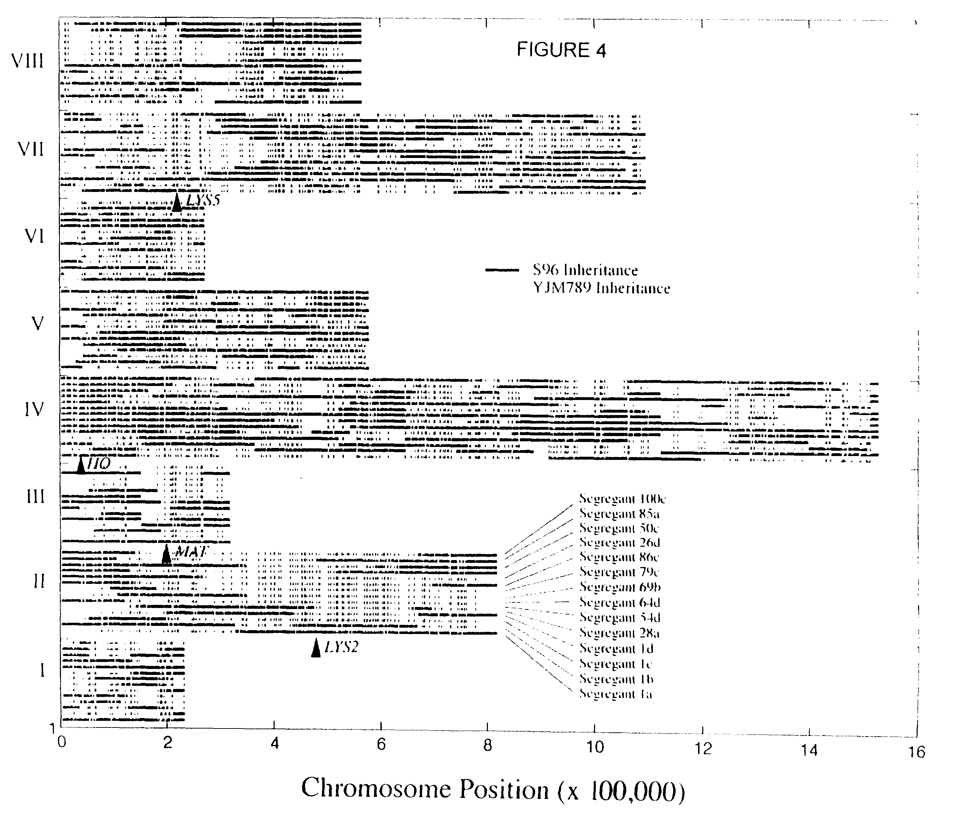

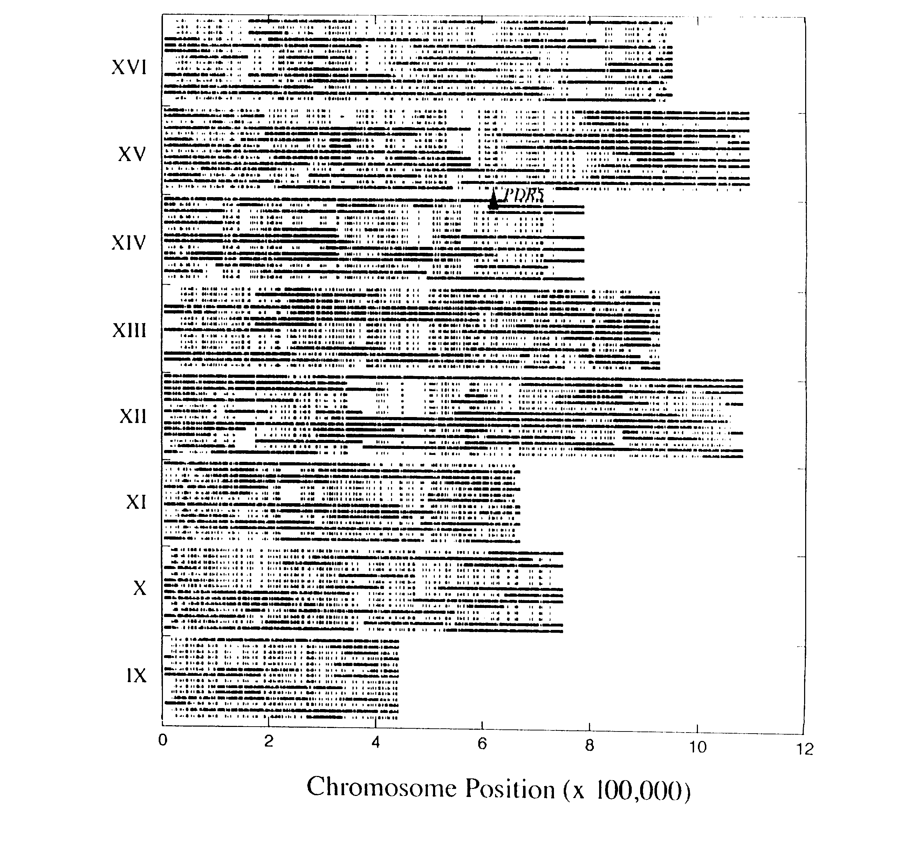

Figure 4 shows the position of all markers (tick marks), the marker's score (color) and the probable parental origin (YJM789 or S96) as a solid bard in pink or dark green below the ticks for ten segregants and as well for the tetrad (segregants 1a to d, described earlier). To determine the probable parental origins, a software routine was written that calculated the locations of recombination breakpoints for each of the segregants for the entire genome using a maximum likelihood method. For each marker, the probability that a signal is from S96 was computed as P(S96)/[P(S96)+P(YJM789)], where P(X) is the probability of observing the signal as described earlier. The maximum likelihood breakpoints were recursively added to each chromosome using these probabilities. The log probability of a breakpoint (and two breakpoints initially and then at chromosome ends) between each pair of markers was tested against the log probability of no breakpoint. The breakpoint(s) that maximized this likelihood were accepted if the lag likelihood was greater than 30. This procedure was repeated for each new sub-interval created by a breakpoint to 500 bp resolution.

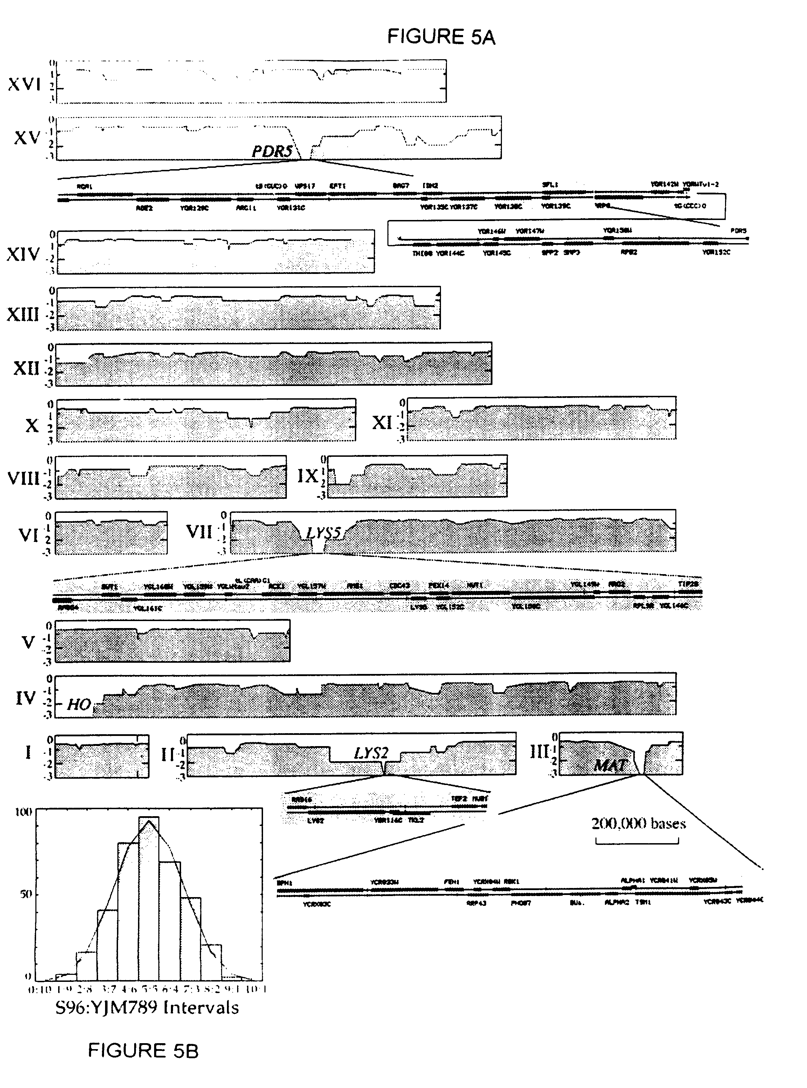

This method allowed aberrantly-segregating markers (caused by gene-conversion events or by other mis-scoring) to be ignored. The number of segregants inheriting a YJM789 or an S96 region was tabulated for every point along the genomic map (Figure 5). The y-axis (log base 10) indicates the probability of random segregation calculated using a binomial distribution. The names and locations of open reading frames inside the intervals with the lowest probability of random segregation (10 out of 10 = (1/2) 10) are shown and are shaded in gray, except for those surrounding *HO.* The empirical and theoretical segregation distributions are shown in the inset. Of the 413 total intervals (continuous chromosomal regions of inheritance across all segregants), 377 were at least 50 cM from all mapped loci. The histogram shows the number of these intervals observed with each S96:YJM789 segregation ratio. The curve is the expected number of intervals for each ratio, according to the binomial distribution.

Only five regions on Chromosomes II, III, IV, VII and XV showed a low probability of random segregation (probability = 0.001 per region). Four of these regions correlate well with the known positions of *LYS2* (Chr II, 469702), *MAT* (Chr III, 198278), *LYS5* (Chr VII, 215281), and *HO* (Chr IV, 46272). The mating type locus *(MAT)* was mapped to 26 kb interval, even though a 59 kb marker gap was located adjacent to this locus and the *LYS2* gene was mapped to a 11 kb region, containing only four candidate genes.

The *HO* locus was mapped to a 96 kb region, but this interval size was reduced to 64.5 kb when the data from the tetrad (whose genotype was known) was included. The cycloheximide sensitivity could be unambiguously mapped to the remaining unassigned 57 kb region on Chromosome XV. These data strongly point to *PDR5* (Chr XV, 619838) as the gene responsible for cycloheximide sensitivity, consistent with previously-observed genetic linkage to the *ade2* and *his3* loci, also located on chromosome XV. To test whether *PDR5* was the actual cause of cycloheximide sensitivity, the *PDR5* gene was deleted in the S96 genetic background and the resulting strain was crossed to YJM789. The deleted strain was unable to complement the cycloheximide sensitivity of YJM789. In addition, when YJM789 array hybridization data were closely examined, a deletion was identified that covered the PDR5 gene, providing further evidence that the loss of this gene was the cause of cycloheximide sensitivity.

The minimum interval (559541 to 616363) based on maximum likelihood calculation of chromosomal breakpoint positions for *cyh* was located just upstream of the *PDR5* gene (619838-624373) due to a chromosomal breakpoint being assigned to a position 3 kb upstream of PDR5 for one segregant (86c). While several markers both upstream and downstream of PDR5 show S96 inheritance for this segregant, markers from *PDR5* itself were of the YJM789 pattern. The misassignment of the chromosome breakpoint is most probably due to a gene conversion event near the breakpoint.

In this work 3808 genetic markers were identified in a natural isolate of *S. cerevisiae* and these markers were used to map five genetic loci in this strain with a resolution ranging from 3 to 35 cM by examining only 10 segregants. The number is low because every marker is informative. It is likely, however, that up to 14 segregants (or more if the trait was non-Mendelian) might need to be examined to have a high probability of only identifying a single region as responsible for a trait.

The set of 3808 markers constitutes about 4.7% of the estimated variation in the strain. At this resolution (approximately 1.0 cM) the map marker density exceeds that of the traditional yeast genetic map (2600 markers) assembled over a period of 40 years. Even more variation might be detected using different arrays designed specifically for the purpose of mapping. Currently arrays can be synthesized at densities of 2.5 X 10⁵ sequences/cm² but improvements in technology promise even denser oligonucleotide arrays. Even at 2.5 x 10⁵ different sequences/cm², a set of six arrays could contain probe pairs for all non-duplicated regions of the yeast genome.

One advantage of the approach described here is simplicity. The entire set of 2560 markers can be scored in one day without amplification steps or enzymatic manipulation. Other methods commonly used for scoring markers involve the prior amplification of the selected fragments of DNA containing the allele beforehand. This same inexpensive direct labeling method employed here could be used to identify and score the inheritance of alleles in metazoans.

The amount of effort expended could also be reduced by using pooling strategies to reduce the number of hybridizations that would need to be performed to map genes. The *MAL* gene was mapped with 45 kb (13 cM) resolution by examining 10 segregants. This interval could be narrowed by examining more segregants but not necessarily by performing more hybridizations. Multiple loci could be mapped with one hybridization of a pooled DNA sample. This adaptation will be important for the analysis of multigenic quantitative trait loci in which DNA from a large number of affected individuals (or strains) will need to be examined to demonstrate linkage.

For the set of experiments reported here, DNA from haploid strains was hybridized to the arrays, effectively making the signal at a position equivalent to what would be observed for the homozygous diploid. However, because high-densty arrays can be used to detect subtle changes in gene expression (as low as 20%), 50% differences in signal at individual probe features in the heterozygote are also detectable. In organisms with short generation times, the sensitivity could be enhanced by performing several backcrosses.

The data presented herein demonstrates that polymorphic strains of a species whose genome sequence is known can be studied using powerful new technologies. The ability to work with polymorphic natural isolates allows researchers to access a virtually unlimited pool of strains or individuals having different interesting heritable characteristics. The analysis of the genetic diversity in populations is likely to be an increasingly important area of research as the number of completed genome sequences grows.

**TABLE I**

| Strain | Genotype | Method of Construction or reference |
|---|---|---|
| S96 | *ho lys5 gal2 SUC2 mal* | Isogenic with S288c |
| YJM145 | *HO gal2 pdr5 MAL SUC2* | |
| YJM789 | *lys2 ho::hisG pdr5 lys2 MA Tα* (isogenic derivative of YJM145) | |
| 1a | *ho MAL pdr5 MA Ta* | segregant of YJM789/S96 |
| 1b | *ho::hisG mal lys5 MA Ta* | segregant of YJM789/S96 |
| 1c | *ho mal lys2 pdr5 MA Tα* | segregant of YJM789/S96 |
| 1d | *ho::hisG MAL lys2 lys5 MATa* | segregant of YJM789/S96 |
| 100c | *ho MAL lys2 pdr5 MA Ta* | segregant of YJM789/S96 |
| 28a | *ho MAL lys2 pdr5 MATα* | segregant of YJM789/S96 |
| 64d | *ho mal lys2 pdr5 MA Ta* | segregant of YJM789/S96 |
| 86c | *ho MAL lys2 pdr5 MA Tα* | segregant of YJM789/S96 |
| 79c | *ho MAL lys2 pdr5 MA Ta* | segregant of YJM789/S96 |
| 69b | *ho MAL lys2 pdr5 MA Tα* | segregant of YJM789/S96 |
| 54d | *ho MAL lys2 pdr5 MA Ta* | segregant of YJM789/S96 |
| 50c | *ho MAL lys2 pdr5 MATα* | segregant of YJM789/S96 |
| 85a | *ho MAL lys2 pdr5 MATa* | segregant of YJM789/S96 |
| 26d | *ho mal lys2 pdr5 MAT*α | segregant of YJM789/S96 |

## Claims

1. A method of detecting allelic variation between two closely related nucleic acid samples, wherein at least a partial nucleotide sequence is known for one of said nucleic acid samples, the method comprising:
obtaining a first nucleic acid sample and a second nucleic acid sample from two closely related sources, wherein the complexity of said nucleic acid samples is at least about 10⁵ nt in length;
fragmenting each of said nucleic acid samples to produce separate pools of fragments having an average size of from 12 to 50 nucleotides in length;
labeling each of said pools of fragments with a detectable label;
hybridizing each of said labeled fragments to a separate microarray comprising at least 10³ oligonucleotides complementary to said known nucleotide sequence, wherein said oligonucleotides are from 12 to 30 nucleotides in length;
detecting the presence of said labeled fragments bound to said microarray;
comparing the signal from said first nucleic acid sample and said second nucleic acid sample, wherein a bimodal distribution between said first and said second nucleic acid samples indicates allelic variation at the genomic locus corresponding to said complementary oligonucleotide.

2. The method according to Claim 1, wherein said closely related nucleic acid samples are two individuals of a single species.

3. The method according to Claim 1, wherein said closely related nucleic acid samples are varieties of a single species.

4. The method according to Claim 1, wherein said closely related nucleic acid samples are two related species.

5. The method according to Claim 1, wherein said nucleic acid samples are a genomic DNA sample.

6. The method according to Claim 1, wherein one of said nucleic acid samples is a pre-determined portion of a genome.

7. The method according to Claim 1, wherein the complexity of said nucleic acid samples is at least about 10⁷ nucleotides.

8. The method according to Claim 1, wherein the complexity of said nucleic acid samples is at least about 10⁸ nucleotides.

9. The method according to Claim 1, wherein said fragments are end-labeled with a detectable label.

10. The method according to Claim 9, wherein said end-label comprises a biotin molecule, which biotin molecule is subsequently bound to an avidin moiety comprising a detectable label.

11. The method according to Claim 1, wherein said detectable label is phycoerythrin.
